# EUROPEAN PATENT SPECIFICATION

(11) **EP 1 543 063 B1**
(45) Date of publication and mention of the grant of the patent: **25.03.2009**
(21) Application number: 03748273.4
(22) Date of filing: 22.09.2003
(51) Int. Cl.: C08G 73/00, C08G 73/02, A61K 47/00, A61K 47/06, A61K 47/16, A61K 47/18, A61K 47/30, A61K 9/00

(54) **DRUG DELIVERY**
ARZNEIMITTELVERABREICHUNG
LIBERATION DE MEDICAMENTS

(30) Priority: 20.09.2002 GB 0221942
(43) Date of publication of application: 22.06.2005
(73) Proprietor: THE SCHOOL OF PHARMACY, UNIVERSITY OF LONDON, London WC1N 1AX (GB)
(72) Inventor: UCHEGBU, Ijeoma, Glasgow G61 3DT (GB); SCHATZLEIN, Andreas, Glasgow G61 3DT (GB); CHENG, Woei, Ping, Glasgow G1 1HE (GB)
(74) Representative: Davies, Isobel Clare
(86) International application number: PCT/GB2003/004036
(87) International publication number: WO 2004/026941

(56) References cited:
- WO-A-02/30468
- WO-A-99/43752
- US-A- 5 338 532
- US-A- 5 681 543
- NÖDING, G.; HEITZ, W.: "Amphiphilic polyethyleneimines based on long-chain alkyl bromide" MACROMOLECULAR CHEMISTRY AND PHYSICS, vol. 199, 1998, pages 1637-1644, XP002268918 cited in the application

## Description

### Field of Invention

This invention relates to the delivery of drugs. In particular, this invention relates to the oral delivery of poorly soluble drugs using novel amphiphilic polymers with both solubilising and absorption enhancing properties.

### Background of Invention

The oral delivery of poorly soluble drugs is usually accomplished with oil based formulations such as microemulsions (Dunn, C.J., Wagstaff, A.J., Perry, C.M., Plosker, G.L., Goa, K.L.,2001, Cyclosporin - An Updated Review of the Pharmacokinetic Properties, Clinical Efficancy and Tolerability of a Microemulsion-Based Formulation Neoral R(1) in Organ Transplantation, Drugs 61: 1957 - 2016; and Porter, C.J.H., Charman, W.N., 2001, In vitro Assessment of Oral Lipid Based Formulations, Advanced Drug Delivery Reviews 50: S127-S147) or low molecular weight surface active agents (BalandraudPieri, N., Queneau P.E., Caroli Bosc, F.X., BertaultPeres, P., Montet, A.M., Durand, A., Montet, J.C. 1997, Effects of Tauroursodeoxycholate Solutions on Cyclosporin and Bioavailability in Rats, Drug Metabolism and Disposition 25: 912-916; Guo, J.X., Ping, Q.N., Chen, Y. 2001, Pharmacokinetic Behaviour of Cyclosporin A in Rabbits by Oral Administration of Lecithin Vesicle and Sandimmun Neoral, International Journal of Pharmaceutics 216: 17-21). Poorly soluble drugs are those drugs that are identified in the British Pharmacopoeia as "practically insoluble" (Medicines Commission, British Pharmacopoeia, The Stationary Office, London, 1998). Such drugs have an aqueous solubility of less than 0.1mg per millilitre of solvent (such as water) at a temperature of about 15°C - 20°C.

Previous attempts to promote oral absorption of poorly soluble drugs such as cyclosporin, have involved the use of oil and/or surfactant (Dunn, C.J., Wagstaff, A.J., Perry, C.M., Plosker, G.L., Goa, K.L.,2001, Cyclosporin - An Updated Review of the Pharmacokinetic Properties Clinical Efficacy and Tolerability of a Microemulsion-Based Formulation Neoral R(1) in Organ Transplantation, Drugs 61: 957 - 2016; and Porter, C.J.H., Charman, S.A., Williams, R.D., Bakalova, M.B., Charman, W.N., 1996, Evaluation of Emulsifiable Glasses for the Oral Administration of the Cyclosporin in Beagle Dogs, International Journal of Pharmaceutics 141: 227-237), bile salt (BaladraudPieri, N., Queneau, P.E., CaroliBosc F.X., BertaultPeres, P., Montet, A.M., Durand, A., Montet, J.C., 1997, Effects of Tauroursodeoxycholate Solutions on Cyclosporin and Bioavailablity in Rats, Drug Metabolism and Disposition 25:912-916), phospholipid based systems (Guo, J.X., Ping, Q.N., Chen, Y., 2001, Pharmacokinetic Behaviour of Cyclosporin A In Rabbits by Oral Administration of Lecithin Vesicle and Sandimmun Neoral, International Journal of Pharmaceutics 21: 17 - 21; and Leigh, M., Hoogevest, P.V., Tiemiessem, H., 2001 Optimising the Oral Bioavailablity of the Poorly Water Soluble Drug Cyclosporin A Using Membrane Lipid Technology, Drug Delivery and Sciences 1: 73-77) or cyclodextrins (Miyake, K., Arima, H., Irie, T., Hirayma, F., Uekama, K., 1999, Enhanced Absorption of Cyclosporin A by Complexation with Dimethyl-Beta-Cyclodextrin in Bile duct-Cannulated and Non-Cannulated Rats, Biological and Pharmaceutical Bulletin 22: 66-72). Although a nanocapsule formed during in-situ polymerisation has also been proposed for cyclosporin delivery, this technique has difficulties in delivering the drug (Bonduelle, S., Carrier, M., Pimienta, C., Benoit, J.P., Lenaerts, B., 1996, Tissue Concentration of Nanoencapsulted Radiolabelled Cyclosporin Following Peroral Delivery in Mice or Opthalmic Application in Rabbits, European Journal of Pharmaceutics and Biopharmaceutics, 42: 31 - 319).

Cyclosporin is a lipophilic immunosuppressant used to treat transplant and autoimmune disease patients. Cyclosporin is poorly soluble in a variety of solvents and is currently administered as micro-emulsion formulation.

Nöding & Heitz (Macromolecular Chemistry and Physics, 199: 1637-1644, 1998) describes the synthesis of polyethylenimine polymers with tertiary nitrogen atoms within the chain in a reaction in which branched polyethylenimines are created by the reaction of polyethylenimine with an alkyl bromide.

US 5681543 discloses polymer complexes for use as diagnostic complexing agents, including polymers derived from tertiary polyethylenimines. The polymers are used to complex metal ions for use in MRI.

US 5338532 discloses dendrimer molecules including dendrimers based on tertiary amines.

WO 02/30468 describes cationic polymers including polyethylenimines that are used to complex nucleic acid for delivering the nucleic acid into cells.

CA 2321200 describes a number of polyethylenimine products having two or more hydrophobic groups for complexing nucleic acid.

It is an object of embodiments of the present invention to obviate or mitigate at least one or more of the aforementioned problems.

It is a further object of embodiments of the present invention to improve delivery of poorly soluble drugs to a recipient.

### Summary of the Invention

Accordingly, in a first aspect, the present invention provides a pharmaceutical composition of a poorly soluble drug and amphiphilic polyethylenimine polymer, wherein the drug has an aqueous solubility of less than 0.1 mg per millilitre of solvent at a temperature of about 15-20°C, wherein the polymer contains hydrophilic substituents that comprise quaternary nitrogen groups and hydrophobic substituents as represented by the formula: 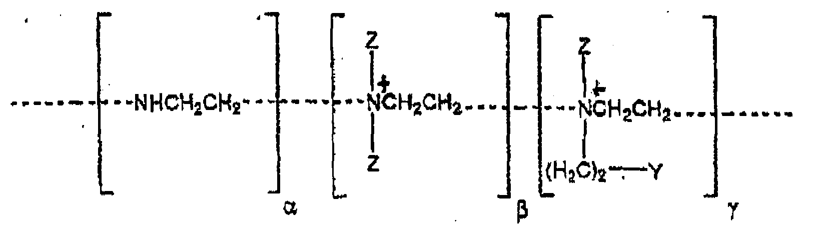 wherein
α is between 0 to 90%;
β is between 0 to 100%;
γ is between 0 to 50%;
and α + β + γ = 100%; and
and further wherein:
each Z group is independently hydrogen or a hydrophobic substituent, wherein the hydrophobic substituent is a linear or branched, substituted or unsubstituted or a cyclic group; and
Y is a hydrophilic substituent.

In a further aspect, the present invention provides the sse of a polyethylenimine polymer as defined above for the preparation of a medicament wherein the polymer is formulated with a poorly soluble drug, wherein the drug has an aqueous solubility of less than 0.1 mg per millilitre of solvent at a temperature of about 15-20°C.

It should be understood that the monomer units identified with α, β and γ may form any arrangement in the polyethylenimine polymer. The arrangement of the α, β and γ units may therefore be random or in a block copolymer form such as αβγαβγαβγ etc. This is identified above by the dashed line between the different monomer units.

The polyethylenimine polymer may be linear or branched.

The ratios for α, β, γ are numerical ratios.

Typically, the Z groups may independently be selected from any of the following hydrophobic substituents: an alkyl, an alkenyl, and alkynyl, an aryl, an acyl, a hydroxy alkyl, a hydroxy acyl, polyethylene glycol or any sugar.

The Z groups may independently be any linear or branched, substituted or unsubstituted, or cyclo form of the following alkyl, alkenyl, alkynyl, aryl, acyl, hydroxy alkyl, hydroxy acyl, polyethylene glycol or any sugar groups: C₁ - C₂₀; C₁ - C₁₂; C₁ - C₆ or C₁.

The Z groups may be C₁ - C₄ linear alkyl groups.

Y may represent any of the following: -NH₂; -NHA; -N⁺R₁R₂R₃; and -N⁺R₁R₂A.

R₁, R₂, or R₃ may be selected from any of the following substituents: an alkyl, an alkenyl, an alkynyl, an aryl, an acyl, a hydroxy alkyl, a hydroxy acyl, polyethylene glycol or any sugar.

R₁, R₂ and R₃ may independently be any linear or branched, substituted or unsubstituted, or cyclo form of the following alkyl, alkenyl, alkynyl, aryl, acyl, hydroxy alkyl, hydroxy acyl, polyethylene glycol or any sugar groups: C₁ - C₂₀; C₁ - C₁₂; C₁ - C₆ or C₁.

Typically, R₁, R₂ and R₃ are C₁ - C₄ linear alkyl groups.

All of R₁, R₂ and R₃ may be CH₃.

Conveniently there may be between 1 and a maximum of 3 R substituents on any single nitrogen. This allows for primary, secondary and tertiary amines.

The groups A may be selected from any of the following linear or branched, substituted or unsubstituted, or cyclo groups: C₁ - C₃₀; C₈- C₂₄; or C₁₂ - C₁₆.

Typically, the groups A may be a linear C₁₂ - C₁₆ alkyl group.

In particular, A may be CH₃(CH₂)₁₅.

The ratio of quaternary ammonium nitrogens to nitrogens of amino groups may be selected from any of the following: 0.01% - 100%; 10% - 90%; 30% - 70%; 40% - 60%; 50% - 90% or 60% - 80%. The preferred range is 40% - 90%. A high proportion of quaternary ammonium groups promotes solubilisation of both the polyethylenimine polymer and a hydrophobic drug.

The parent polyethylenimine compound used to make the polyethylenimine polymer may have an average molecular weight of about 2 - 50kD, or more particularly, of about 10 - 25 kD.

The polyethylene polymer may have an average molecular weight of about 10 - 25 kD.

The polyethylenimine polymer may produce hydrophobic domains. Hydrophobic domains are areas of the molecule's self-assembly where hydrophobic compounds or compounds which are poorly soluble in water are able to reside and thus become solubilised with an aqueous disperse phase. The level of hydrophobic modification may be from 0.01 - 50%, 0.1 - 20% or 1 - 10% of amino groups. The preferred level of hydrophobic modification is 1 - 10% of amino groups.

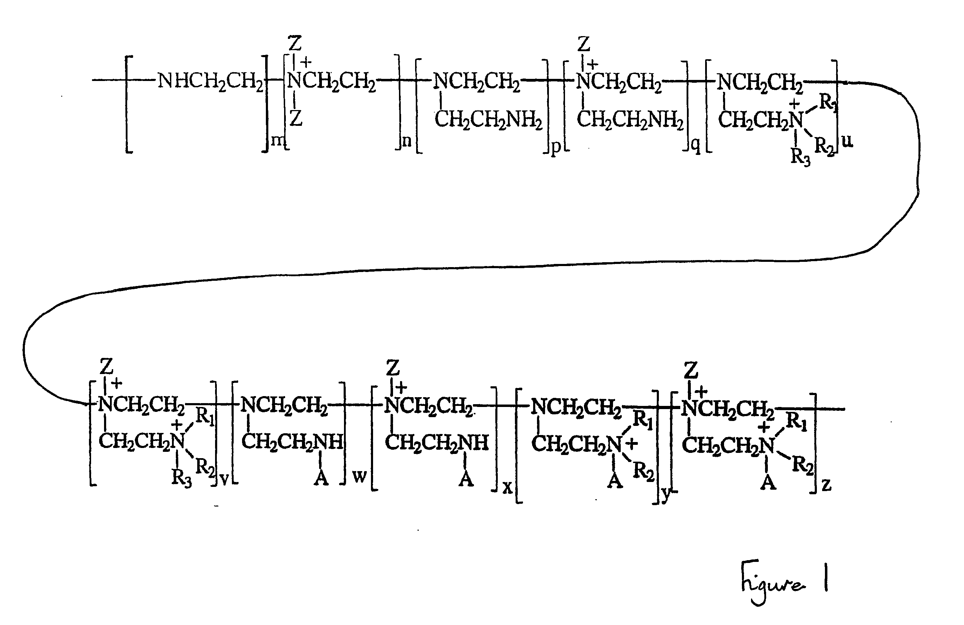

All possible monomeric subunits in accordance with the structure as defined in formula I are shown in Figure 1:
wherein
m is between 0 - 90 %;
n is between 0 - 100 %;
p is between 0 - 50 %;
q is between 0 - 50 %;
u is between 0 - 50 %;
v is between 0 - 50 %;
w is between 0 - 20 %;
x is between 0 - 20 %;
y is between 0 - 20 %; and
z is between 0 - 20 %;
wherein, m + n + p + q + u + v + w + x + y + z = 100%; and
A, R₁, R₂, R₃ and Z are as defined above.

It should be appreciated that the monomer units m, n, p, q, u, v, w, x, y and z may be arranged in any order.

The ratios for m, n, p, q, u, v, w, x, y and z are numerical ratios.

Typically, if m = 0% then n is not equal to 0%.

Typically, if n = 0% then m is not equal to 0 %.

Typically, if p = 0% then q + u + v + w + x + y + z does not equal 0%.

Typically, if q = 0% then p + u + v + w + x + y + z does not equal 0%.

Typically, if u = 0% then p + q + v + w + x + y + z does not equal 0%.

Typically, if v = 0% then p + q + u + w + x + y + z does not equal 0%.

Typically, if w = 0% then x + y + z + n does not equal 0%.

Typically, if x = 0% then w + y + z + n does not equal 0%.

Typically, if y = 0% then w + x + z + n does not equal 0%.

Typically, if z = 0% then w + x + y + n does not equal zero.

Conveniently, m + n lies between 50 to 100%.

Conveniently, p + q + u + v lies between 20 to 50%.

Conveniently, w + x + y + z lies between 0.01 to 10%.

It is possible that polyethylenime may be linear (n=100) or branched as shown in Figure 1. If n = 0%, however, then m must be equal to a value greater than 0% as this allows for the branched material with no backbone quaternisation on erstwhile secondary amines.

It is possible that p, q, u, v, w, x, y or z may be equal to 0%. However, the sum total of p, q, u, v, w, x, y and z may be equal to a value greater than 0%, as this allows for the branched compound to be included.

Alternatively, w, x, y or z may be equal to 0%. However, the sum total of w, x, y or z may not be equal to 0%. This allows for a hydrohobically substituted branched compound.

Typically, m + n = 60%, w + x + y + z = 6%, and p + q + u + v = 34%. Using these ranges defines the quaternary ammonium cetyl polyethylenimine found in the Example Section of the present application.

According to a second aspect of the present invention there is provided a method of forming a polyethylenimine polymer according to the first aspect by reacting a polyethylenimine compound formed from the polymerisation of ethylenimine with a first organo halide to form an organo side chain on the polyethylenimine compound, and then a second organo halide to react with an amino group on the polyethyleneimine compound.

The polyethylenimine used may be branched or linear.

Branched polyethylenimine may be prepared by the acid catalysed polymerisation of, for example, aziridine (ethyleneimine) (Dick, C.R., Ham, G.E., J. Macromol. Sci. 1970, A4, 1301-1314; von Harpe, A., Petersen, H., Li, Y., Kissel, T., J. Control. Rel. 2000, 69, 309-332). Linear polymers may be prepared by controlling the conditions of polyethylenimine polymerisation (Zhuk, D.S., Gembitsky, P.A., Alexandrovich, A.I., US Patent No. 4,032,480).

The first organo halide may be any linear or branched, substituted or unsubsituted, or cyclo form of any alkyl, alkenyl, alkynyl, aryl or acyl halide or any hydrophilic halide. The halide may be any of fluoride, chloride, bromide or iodide.

The organo group of the first organo halide may be selected from any of the following linear or branched, substituted or unsubstituted, or cyclo groups: C₁ - C₃₀; C₈ - C₂₄; or C₁₂ - C₁₆.

Typically, the first organo halide is a linear C₁₂ - C₁₆ alkyl halide.

In particular, the first organo halide may be cetyl bromide (e.g. CH₃(CH₂)₁₅ Br).

The second organo halide may be any alkyl, alkenyl, alkynyl, aryl or acyl halide or any hydrophilic halide. The halide may be any of fluoride, chloride, bromide or iodide.

The organo group of the second organo halide may be selected' from any of the following linear or branched, substituted or unsubstituted, or cyclo groups: C₁ - C₂₀; C₁ - C₆: or C₁.

Typically, the second organo halide is a linear C₁ - C₆ alkyl halide. In particular, the second organo halide may be methyl iodide.

The polyethylenimine compound and first organo halide may be mixed in an organic solvent such as tetrahydrofuran, which may then be refluxed. The refluxing may occur in an alcoholic solution of, for example, sodium hydroxide. Cetyl polyethylenimine may then be isolated and may then be reacted with the second organo halide.

The second organo halide may be added in the presence of, for example, a metal hydroxide (e.g. sodium hydroxide), a metal halide (e.g. sodium iodide) and an alcohol (e.g. methanol).

The polyethylenimine polymer may then be obtained by washing, dialysis and using an ion exchange column.

Further quaternisation may be obtained by adding more of the second organo halide.

The formed polyethylenimine polymer may be that as represented in Figure 1.

It is also possible to prepare a substituted linear polyethylenimine with the end nitrogens protected, subsequently deprotect the terminal amines and then attach this substituted linear polyethylenimine to the branched molecule and follow the whole conjugation step with a quaternary ammonium step.

Pharmaceutically acceptable carriers are well known to those skilled in the art and include, but are not limited to, 0.1 M and preferably 0.05 M phosphate buffer or 0.9% w/v saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Typically, the ratio of polyethylenimine polymer to pharmaceutically acceptable carrier ranges from any of the following: 0.0001 - 100 w.v., 0. 005 - 50 w.v.; 0.001 - 30 w.v.; 0.001 - 10 w.v.; or 0.01 - 1 w.v.

The drug may be poorly soluble in aqueous solvents such as water. The drug may be administered to a patient as a solution or a particulate formulation.

The drug may be selected from any of the following: cyclosporin; steroids such as prednisolone, oestradiol, testosterone; drugs with multicyclic ring structures which lack polar groups such as paclitaxel; and drugs such as etoposide.

Typically, the ratio of the polyethylenimine polymer to the drug may be selected from any of the following: 0.001 - 100%; 0.1 - 100%; 1 - 100%; 10 - 90%; 30 - 70%.

The pharmaceutical composition may also comprise a pharmaceutically acceptable carrier.

Typically, the ratio of polyethylenimine polymer to drug to pharmaceutically acceptable carrier may be in the range of 5 - 20mg : 0.5 - 5mg : 0.5 - 5mL or 5 - 20mg : 0.5 - 5mg : 0.5 - 5g. In particular, the ratio of polyethylenimine polymer to drug to pharmaceutically acceptable carrier may be about 10mg:2mg:1mL or about 10mg:2mg:2g.

The pharmaceutical composition may be in the form of any of the following: tablets, suppositories, liquid capsule, powder form, or a form suitable for pulmonary delivery.

When tablets are used for oral administration, typically used carriers include sucrose, lactose, mannitol, maltitol, dextran, corn starch, typical lubricants such as magnesium stearate, preservatives such as paraben, sorbin, antioxidants such as ascorbic acid, α-tocopheral, cysteine, disintegrators or binders. When administered orally as capsules, effective diluents include lactose and dry corn starch. A liquid for oral use includes syrup, suspension, solution and emulsion, which may contain a typical inert diluent used in this field, such as water. In addition, sweeteners or flavours may be contained.

Suppositories may be prepared by admixing the compounds of the present invention with a suitable non-irritative excipient such as those that are solid at normal temperature but become liquid at the temperature in the intestine and melt in rectum to release the active ingredient, such as cocoa butter and polyethylene glycols.

The dose of the polymer can be determined on age, body weight, administration time, administration method, combination of drugs, the level of condition of which a patient is undergoing therapy, and other factors. While the daily does may vary depending on the conditions and body weight of patients, the species of active ingredient, and administration route, in the case of oral use, the daily does may be about 0.1 - 100 mg/person/day, preferably 0.5 - 30 mg/person/day.

### Brief Description of the Drawings

Embodiments of the present invention will now be described, by way of example only, with reference to the accompanying drawings in which:
Figure 1 is a representation of a polyethyleneimine polymer formed according to the present invention; and
Figure 2 is a Transmission Electron Microscopy (TEM) image of quaternary ammonium cetyl polyethyleneimine (QCPEI2) and cyclosporin nanoparticles.

### Examples

### Example 1 - Synthesis of Quaternary Ammonium Cetyl Polyethylenimine (QCPEI)

Alkylation of polyethylenimine was carried out according to a previously reported method (Noding, G., Heitz, W., 1998, Amphiphilic Polyethylenimines Based on Long-Chain Alkyl Bromide Macromolecular Chemistry and Physics 199: 637 - 1644). Briefly, polyethylenimine (M_{w} = 25kD, 5g) was alkylated by refluxing with cetyl bromide (1.8g) and tetrahydrofuran (50ml) for 48 hours, followed by the addition of an alcoholic solution of sodium hydroxide (4.8g in 25ml methanol), and a further reflux period of 24 hours. Sodium bromide was removed by filtration and the product isolated by evaporation of the solvent, exhaustive dialysis and freeze-drying. 0.6g of cetyl polyethylenimine was then quaternised by reaction with methyl iodide (2.6ml) in the presence of sodium hydroxide (0.23g), sodium iodide (0.28g) and methanol (100ml) for 3 hours at 36°C. The product was isolated by precipitation in ether (400ml), washing with ethanol, exhaustive dialysis of an ethanolic solution and elution through an ion exchange column to isolate the hydrochloride salt.

A yellow cotton wool like solid which is the quaternary ammonium cetyl polyethyleneimine (QCPEI1) was obtained on freeze drying.

A further quaternisation of quaternary ammonium cetyl polyethyleneimine (QCPEI1) produced a doubly quaternerised compound, i.e. di-quaternary ammonium cetyl polyethyleneimine (QCPEI2).

### Characterisation of Quaternary Ammonium Cetyl Polyethylenimine

¹H NMR and ¹H correlation spectroscopy as well as ¹³C NMR experiments (Bruker, AMX 400 MHz spectrometer, Bruker Instruments UK) were carried out on the quaternary cetyl polyethyleneimine in deuterated methanol. Elemental analysis was carried out on the products using a Perkin Elmer 2400 analyser.

### Polymer Aggregation

The aggregation of an aqueous solution of the polymers was studied using a pyrene probe for hydrophobic domains (see Kalyanasundaram, K., Thomas, J.K., 1977, Environmental Effects on the Vibronic Band Intensities in Pyrene Monomer Fluorescence and the Application to Studies of Micellar Systems, Journal of the American Chemical Society 99: 2039 - 2044). Fluorescence scans (excitation = 340nm) were performed on various concentrations of the polymer dissolved in an aqueous pyrene solution (2µM). The ratio of the intensity of the third and first peaks (I₃/I₁) was used to assess the hydrophobicity of the pyrene environment which is an indirect probe for polymer association.

Polymer aggregation was also assessed by recording the hypsochromic shift in the UV absorption spectrum of methyl orange (Lieske, A., Jaeger, W., 1999, Block Copolymers Containing Polysoap Blocks, Tenside Surfactants Detergents 36: 155 - 161) in 25µM in 0.02M borate buffer when encapsulated within a hydrophobic environment. UV absorption scans (300 - 600nm) were performed on various concentrations of the polymer dissolved in the methyl orange-borate solution and the wavelength of maximum absorbance noted.

**TABLE 1: Quaternary ammonium cetyl polyethyleneimine (QCPEI1) aggregation in aqueous solution as measured by the increase in (I₃/I₁) ratio in the pyrene fluorescence and by the hypsochromic shift in the methyl orange spectra**

| **QCPEI1 I3/I1 ratio (QCPEI1 concentration in mg mL⁻¹)** | **QCPEI1 Methyl Orange wavelength of maximum absorbance (QCPEI1 concentration in mg mL⁻¹)** | **QCPEI2 I3/I1 ratio (QCPEI2 concentration in mg mL⁻¹)** | **QCPEI2 Methyl Orange wavelength of maximum absorbance (QCPEI2 concentration in mg mL⁻¹)** |
|---|---|---|---|
| 0.64 (0) | 465(0) | 0.61 (0) | 465 (0) |
| 0.88 (0.87) | 450 (0.50) | 0.823 (0.81) | 456 (0.55) |
| 0.89 (1.73) | 452 (1.52) | 0.862 (1.621) | 450 (1.63) |
| 0.92 (3.73) | 452 (3.73) | 0.871 (3.24) | 458 (3.70) |
| 0.98 (7.04) | 454 (7.80) | 0.853 (4.37) | 455 (7.85) |
| | | 0.926 (6.49) | 456 (14.25) |

The synthesis of the cetyl polyethylenimine was confirmed by a proton NMR and assignments were made as follows:
δ = 0.87 = CH₃ (cetyl), δ1.25 = CH₂ (cetyl), δ1.45 = CH₂ - N (cetyl), 52.7 - 2.8 = CH₂ - N (cetyl and polyethylenimine). Quaternisation of cetyl polyethylenimine to produce quaternary ammonium cetyl polyethylenimine was confirmed by ¹³C NMR: δ14.6 = CH₃(cetyl), δ23.9 = CH₂(cetyl), δ52.5 and 54.8 = CH₃(CH₃N⁺), 558.8 and 63.5 = CH₂N and CH₂N+(polyethylenimine) and ¹H NMR - δ0.90 = CH₃ (cetyl), δ1.3 = CH₂ (cetyl), 51.47 = CH₂ (cetyl), δ1.85 = CH₂ - N (cetyl), 52.5 - 4.7 = CH₂N, CH₂N⁺ and CH₃N⁺.

The yields of cetyl polyethylenimine, quaternary polyethyleneimine (QCPEI1) and di-quaternary cetyl polyethyleneimine (QCPEI1) were 67%, 85% and 46%, respectively.

The degree of cetylation was found to be 5.2% of all amine groups using elemental analysis data. The degree of conversion of amines to quaternary ammonium moieties was approximately 64% for quaternary cetyl polyethylenimine and 81% for di-quaternary cetyl polyethylenimine.

Both quaternary ammonium polymers aggregate to produce hydrophobic domains in aqueous solution (See Table 1). This is shown by the increase in the I3/I1 values and also by the shift to a lower wavelength of the methyl orange peak. These hydrophobic domains serve to solubilise poorly aqueous soluble (hydrophobic) drugs such as cyclosporin; in the case of the less quaternised variant - QCPEI1 which forms a clear micellar liquid with cyclosporin, when freshly prepared (Table 1), effectively encapsulating cyclosporin within the hydrophobic domains.

### Example 2 - Preparation of Quaternary Cetyl Polyethylenimine - Cyclosporin Formulations

Quaternary cetyl polyethylenimine polymers were dissolved by probe sonication on ice (Soniprep Instruments, UK) followed by the addition of cyclosporin, which was incorporated into the polymer solution by probe sonication. Formulations were stored for up to 13 days and observed for particle formation. Particulate formations were sized by photon correlation spectroscopy, imaged by both transmission electron microscopy (TEM) with negative staining (see Wang, W., Tetley, L., Uchegbu, I.F., 2001. The Level of Hydrophobic Substitution and the Molecular Weight of Amphiphilic Poly-L-Lysine-based Polymers Strongly Affects Their Assembly into Polymeric Bilayer Vesicles, Journal of Colloid and Interface Science 237: 200-207) and freeze fracture electron microscopy (see Uchegbu, I.F., Schatzlein, A.G., Tetley, L., Gray, A.I., Sludden, J., Siddique, S., Mosha, E., 1998, Polymeric Chitosan - Based Vesicles for Drug Delivery, Journal of Pharmacy and Pharmacology 50: 453-458). Clear micellar formulations were filtered with a 0.45µm filter and the filtered formulations assayed by HPLC using a reverse phase Waters Spherisorb ODS column (25cm x 4.6mm), eluted with a water, acetonitrile tert-butyl methyl ether, orthophosphoric acid (350:600:50:1). Detection was by UV(λ=210nm).

**Table 2: QCPEI-cyclosporin formulations**

| Formulation | Initial Appearance | Initial Mean Particle Size (nm) | % Recovery of cyclosporin from micellar solutions(a) | | Mean Particle Size (nm) | |
|---|---|---|---|---|---|---|
| | | | Freshly prepared (mean ± s.d.) | After storage (2-8°C) for 90 days (mean ± s.d.) | Storage (2-8°C) for 4 days followed by exposure to room temperature for 15 min | Storage (2-8°C) for 3 days followed by exposure to 37°C for 15 min |
| QCPEI1 | Clear liquid | - | 78.7±8.14 (n=3) | 93.3±6.60 (n=4) | 558 (n=3) | 608 (n=6) |
| QCPEI2 | Colloidal | 310 (n=4) | - | - | 377(n=1) | 512(n=3) |

| | | | | | | |
|---|---|---|---|---|---|---|
| (a) Initial Concentration = 2mg mL⁻¹ n Denotes number of formulations assayed. | | | | | | |

[In Table 2 the blank boxes (represented with a "-") represent particulate formulations, which cannot be assayed in the same way as micellar formulations].

As shown in Table 1 both quaternary ammonium polymers (i.e. QCPEI1 and QCPEI2) aggregate to produce hydrophobic domains in aqueous solutions. These hydrophobic domains serve to solubilise cyclosporin. In the case of the less quaternerised variant - QCPEI1 forms a clear micellar liquid with cyclosporin, when freshly prepared, effectively encapsulating cyclosporin within hydrophobic domains. However, as shown in Table 2, the polymer exhibits a lower critical solution temperature and becomes less hydrated with increase in temperature resulting in aggregation of the polymeric micelles to form nanoparticles. Furthermore, Table 2 shows storage of QCPEI1 at refrigeration temperature preserved the micellar formulation. The micellar formulation is preserved as analysis of the optically clear samples after storage for 90 days shows that there is no precipation of cyclosporin.

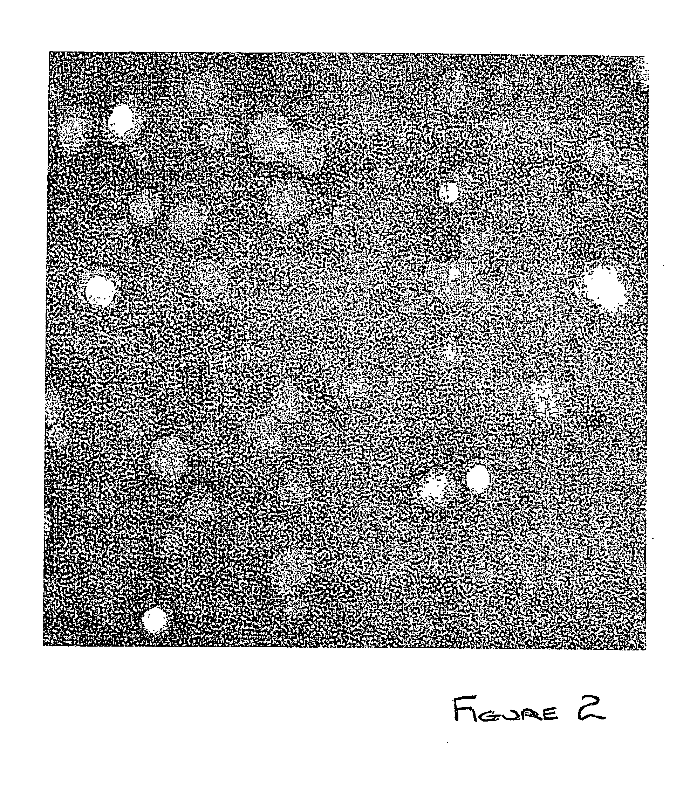

In contrast to QCPEI1, the doubly quaternarised compound QCPEI2, which is less water soluble than QCPEI1 initially formed stable nanoparticles with cyclosporin. Figure 2 shows that the double quaternarised compound (QCPEI2) does not form micelles with cyclosporin. The size bar shows that the aggregates formed are too large to be micelles although the image could show an aggregate of lots of micelles. These will still be technically nanoparticles as the formulation is not optically clear.

Although the polymer forms micelles within which cyclosporin is solubilised, the polymer exhibits a lower critical solution temperature and becomes less hydrated with increase in temperature resulting in aggregation of the polymeric micelles to form nanoparticles after exposure to elevated temperatures (i.e. removal from the fridge, Table 2). However, storage of QCPEI1 at refrigeration temperature preserved the micellar formulation (Table 2) and there was no conversion of the micelles into nanoparticles. In contrast to QCPEI1, the doubly quaternised compound QCPEI2, which is less water soluble than QCPEI1, initially formed stable nanoparticles with cyclosporin (Figure 2, Table 2) and does not form the micelles with cyclosporin.

### Example 3 - Oral Administration of Quaternary Cetyl Polyethylenimine-Cyclosporin Formulations

Groups of male Wistar rats (n=4 i.e. the group size, weight = 200 - 220g) were fasted for 12 hours before dosing and subsequently dosed intragastrically (10mg kg⁻¹) with an optically clear quaternary cetyl polyethylenimine (QCPEI1) - cyclosporin formulation (10:2); a particulate quaternary cetyl polyethylenimine (QCPEI2) - Cyclosporin (10:2) formulation; Neoral (Registered Trademark) or water. Neoral is a microemulsion formulation of cyclosporin manufactured and marketed by Novartis.

Blood was taken from the tail vein of these anaesthetised rats at 1 hour, 4 hours and 24 hours after dosing. Plasma was separated by centrifugation at 1000g and stored at -20°C until analysis could be performed on the samples. Cyclosporin was measured in the plasma samples using a monoclonal antibody radioimmunoassay kit (Cyclo-Trac SP-Whole Body Radioimmunoassay Kit) supplied by Diasorin, UK.

**Table 3: Blood Levels Following Oral Cyclosporin Dosing**

| **Time** | Formulations ngL⁻¹ of cyclosporin in blood | | |
|---|---|---|---|
| | Neoral ^{®} | QCPEI1 | QCPEI2 |
| **1h** | 1525±267* | 583±284 | 748±482 |
| **4h** | 1521±163 | 1179±360 | 1387±539 |
| **24h** | 346±37 | 315±95 | 295±45 |

| | | | |
|---|---|---|---|
| *** = statistically significant difference between groups at the same time point (p<0.05)** | | | |

The oral QCPEI1 formulations were well tolerated in rats with no gross adverse events recorded. Plasma levels at the 4 hour time point from the oil free QCPEI formulations were indistinguishable from peak levels obtained using Neoral (Registered Trademark), although Neoral (Registered Trademark) was absorbed faster than the QCPEI formulations shown in Table 3. The amphiphilic polyethyleneimine polymer therefore promotes the absorption of a poorly soluble drug such as cyclosporin.

Within the 37°C environment of the gut lumen it is assumed, although not wishing to be bound by theory, that the narrow particle formulation prevails for both polymers and that these nanoparticles experience the gradual loss of cationic micellar aggregates still encapsulating their hydrophobic payload. As cationic polymers are known to facilitate transport across epithelial membranes and across cell membranes, these micellar aggregates may also facilitate the intestinal absorption of cyclosporin. The disassociation of the nanoparticle into single micellar aggregates results in the delayed absorption when compared to the oil containing formulation.

### Example 4 - Oral Delivery of Cyclosporin 2

This Example examines the effect of intermediate and low molecular weight quaternary ammonium hexadecyl polyethylenimine on the oral delivery of cyclosporine A.

### Materials

Polyethylenimine (Mw = 10kD) was supplied by Polysciences, UK. Polyethylenimine (Mw = 1.8kD), hexadecyl bromide, methyl iodide and sodium iodide were all obtained from Sigma-Aldrich, Co., UK. Ethanol, diethyl ether and tetrahydrofuran were supplied by the Department of Pure and Applied Chemistry, University of Strathclyde.

### Methods

Intermediate molecular weight quaternary ammonium cetyl PEI with two different levels of quaternary ammonium modification (Q1₁₀ and Q2₁₀) were synthesised by reacting polyethylenimine (PEI, Mw = 10kD) with both cetyl bromide and methyl iodide as described for QCPEI1 and QCPEI2 respectively in Example 1. Low molecular weight quaternary ammonium cetyl PEI with a high level of quaternary ammonium modification (Q2_{1.8}) was synthesised by reaction of PEI (Mw = 1.8kD) with both cetyl bromide and methyl iodide as described for QCPEI2 in Example 1. Q1₁₀, Q2₁₀, and Q2_{1.8} cyclosporine (2mg mL⁻¹) formulations, each containing 10mg mL⁻¹ of the respective amphiphilic PEI were prepared as described in Example 2.

Male Wistar rats (mean weight = XXg [WPC PLEASE COMPLETE], n = 4) were dosed orally with QCPEI1, Q1₁₀, Q2₁₀ or Neoral formulations of cyclosporine (7.5mg kg⁻¹). Blood was then sampled at various time intervals and cyclosporine analysed in the sampled blood using the radioimmunoassay procedure described in Example 3. In a separate experiment male Wistar rats (mean weight = XXg [WPC PLEASE COMPLETE], n = 4) were dosed orally with Q2₁₀, Q2_{1.8}, or Neoral formulations of cyclosporine (10mg kg⁻¹). A further group was dosed with a dispersion of cyclosporine (10mg kg⁻¹) in water which was shaken just prior to administration. Blood was sampled from these 4 groups of animals at various time intervals and cyclosporine analysed in blood using the radioimmunoassay procedure described in Example 3.

### Results

**Table 4: Blood levels of cyclosporine after dosing animals orally with 7.5mg Kg⁻¹ cyclosporine**

| Formulation | Blood levels (ng mL⁻¹, n = 4, mean ± s.d.) | | |
|---|---|---|---|
| | 1h | 4h | 24h |
| Q1₁₀ | 615 ± 351* | 854 ± 376 | 73 ± 38 |
| Q2₁₀ | 1050 ± 456 | 1163 ± 326 | 95 ± 19 |
| QCPEI1 | 576 ± 320* | 799 ± 481 | 84 ± 44 |
| Neoral | 1496 ± 447 | 989 ± 301 | 150 ± 68 |

| | | | |
|---|---|---|---|
| *Statistically significantly different from Neoral (p < 0.05) | | | |

**Table 5: Blood levels of cyclosporine after dosing animals orally with 10mg Kg⁻¹ cyclosporine**

| Formulation | Blood levels (ng mL⁻¹, n = 4, mean ± s.d.) | | |
|---|---|---|---|
| | 1h | 4h | 24h |
| Q2_{1.8} | 889 ± 336* | 1677 ± 840 | 461 ± 153^{#} |
| Q2₁₀ | 1213 ± 196* # | 1865 ± 516^{#} | 565 ± 115^{#} |
| Cyclosporine dispersion in water | 439 ± 345* | 617 ± 277* | 88 ± 43 |
| Neoral | 2026 ± 209^{#} | 1915 ± 158^{#} | 475 ± 133^{#} |

| | | | |
|---|---|---|---|
| *Statistically significantly different from Neoral (p < 0.05). ^{#}statistically significantly different from cyclosporine dispersion in water. | | | |

### Comment on Results

At the 7.5mg kg-1 dose level Q2₁₀ had an equivalent bioavailability with Neoral while Q1₁₀ and QCPEI1 delivered less cyclosporine via the oral route after 1h when compared to Neoral, although cyclosporine levels equivalent to Neoral were delivered at the 4h and 24h time points by both Q1₁₀ and QCPEI1.

At the 10mg kg-1 dose level, all formulations delivered less cyclosporine than Neoral at the 1h time point although Q2₁₀ improved the absorption of cyclosporine when compared to cyclosporine dispersion in water. At the 4h time point both Q2₁₀ and Q2_{1.8} were bioequivalent with Neoral whereas due to the high standard deviations obtained with Q2_{1.8}, this formulation was statistically indistinguishable from the cyclosporine dispersion in water. At the 24h time point all formulations resulted in a greater absorption of cyclosporine when compared to the cyclosporine dispersion in water.

It is clear that polyethylenimine amphiphiles are able to promote the absorption of cyclosporine.

### Example 5: Stability of Cyclosporin Solutions

This Example relates to assessing the stability of quaternary ammonium polyethylenimine - cyclosporine formulations.

### Materials

Polyethylenimine (Mw = 10kD) was supplied by Polysciences, UK. Polyethylenimine (MW = 25kD), hexadecyl bromide, methyl iodide and sodium iodide were all obtained from Sigma-Aldrich, Co., UK. Ethanol, diethyl ether and tetrahydrofuran were supplied by the Department of Pure and Applied Chemistry, University of Strathclyde.

### Methods

Q1₁₀ was synthesised by reacting polyethylenimine (PEI, Mw = 10kD) with both cetyl bromide and methyl iodide as described for QCPEI1 in Example 1. QCPEI1 was also synthesised as described in Example 1. Q1₁₀ and QCPEI1 Formulations of cyclosporine (2mg mL⁻¹) containing 10mg mL⁻¹ of the amphiphilic PEIs were prepared as described in Example 2.

Formulations were then stored in stoppered glass containers at refrigeration temperature (2 - 8ºC). At various time intervals aliquots were sampled, filtered through a 0.45µm filter and analysed by high performance liquid chromatography (HPLC). Filtered cyclosporine samples (20µL) dissolved in acetonitrile, water (1: 1) were injected onto a Waters Spherisorb 5µm, 4.6mm X 250mm column (Waters Instruments, UK) maintained at 80°C with a Jones Chromatography Column Heater model 7971 by means of a Waters 717 autosampler and a Waters 515 isocratic pump. The mobile phase was acetonitrile: water: tert-butylmethyl-ether: phosphoric acid (600:350:50:1) at a flow rate of 1.2mL min⁻¹. Peak detection was via a Waters 486 variable wavelength UV detector with the wavelength set at 210nm and data was collected using a Waters 746 data module. A standard curve was prepared using solutions of the drug (1 - 10µg mL⁻¹).

### Results

| Time Point (days) | QCPEI1 | Q1₁₀ |
|---|---|---|
| 0 | 78.7 ± 8.1 | 80.7 ± 17.7 |
| 7 | 84.6 ± 3.1 | 74.0 ± 8.1 |
| 41 | 93.7 ± 8.8 | 81.9 ± 3.6 |
| 109 | 91.0 ± 6.3 | 79.0 ± 0.6 |
| 181 | 84.4 ± 2.9 | 82.0 ± 2.3 |
| 281 | 89.4 ± 0.42 | 79.0 ± 1.4 |

### Comment on Results

Over a 9 month period the level of cyclosporine recovered from amphiphilic PEI formulations Q1₁₀ and QCPEI1 did not differ appreciably from the original levels, indicating that these formulations were stable when stored for 9 months at refrigeration temperatures.

## Claims

1. A pharmaceutical composition of a poorly soluble drug and amphiphilic polyethylenimine polymer, wherein the drug has an aqueous solubility of less than 0.1 mg per millilitre of solvent at a temperature of about 15-20°C, wherein the polymer contains hydrophilic substituents that comprise quaternary nitrogen groups and hydrophobic substituents as represented by the formula: 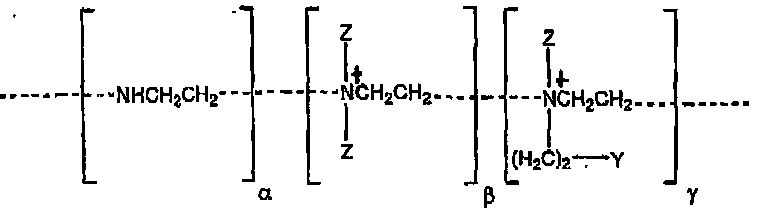 wherein
α is between 0 to 90%;
β is between 0 to 100%;
γ is between 0 to 50%;
and α + β + γ = 100%; and
and further wherein:
each Z group is independently hydrogen or a hydrophobic substituent, wherein the hydrophobic substituent is a linear or branched, substituted or unsubstituted or a cyclic group; and
Y is a hydrophilic substituent.

2. A pharmaceutical composition according to claim 1, wherein:
(a) the monomer units identified with α, β and γ form any arrangement in the polyethylenimine polymer;
(b) the arrangement of the α, β and γ units are random or in a block copolymer form.

3. A pharmaceutical composition according to claim 1 or claim 2, wherein the polyethylenimine polymer is linear or branched.

4. A pharmaceutical composition according to any one of preceding claims, wherein in the polyethylenimine polymer:
(a) at least one of the Z groups is selected from: an alkyl group, an alkenyl group, and alkynyl group, an aryl group or an acyl group; or
(b) at least one of the Z groups is selected a C₁-C₂₀ group, a C₁-C₁₂ group, a C₁-C₆ group or a C₁ group; or
(c) at least one of the Z groups is a C₁-C₄ linear alkyl group.

5. A pharmaceutical composition according to any one of the preceding claims, wherein in the polyethylenimine polymer, Y represents -NH₂, -NHA, -N⁺R₁R₂R₃ or -N⁺R₁R₂A, and wherein each of R₁, R₂, or R₃ is independently a linear or branched, substituted or unsubstituted, or cyclo group selected from: alkyl, alkenyl, alkynyl, aryl, acyl, hydroxy alkyl, hydroxy acyl, polyethylene glycol or a sugar; and A is a C₁-C₃₀ linear or branched, substituted or unsubstituted or cyclo group.

6. A pharmaceutical composition according to claim 5, wherein in the polyethylenimine polymer, there are between 1 and 3 R₁₋₃ substituents on any single nitrogen.

7. A pharmaceutical composition according to claim 5 or claim 6, wherein:
(a) each of R₁, R₂ and R₃ is independently a C₁-C₂₀ group, a C₁-C₁₂ group, a C₁-C₆ group or a C₁ group; or
(b) each of R₁, R₂ and R₃ is a C₁-C₄ linear alkyl group; or
(c) each of R₁, R₂ and R₃ is CH₃.

8. A pharmaceutical composition according to claim 5, wherein in the polyethylenimine polymer:
(a) the A group is a linear or branched, substituted or unsubstituted, or cyclo groups selected from a C₁-C₃₀, a C₈-C₂₄ group or a C₁₂-C₁₆ group; or
(b) the A group is a linear C₁₂-C₁₆ alkyl group; or
(c) the A group is CH₃(CH₂)₁₅.

9. A pharmaceutical composition according to any one of the preceding claims, wherein in the polyethylenimine polymer, the ratio of quaternary ammonium nitrogens to nitrogens of amino groups is selected from: 0.01% - 100%; 10% - 90%; 30% - 70%; 40% - 60%; 50% - 90%; 60% - 80% or 40% - 90%.

10. A pharmaceutical composition according to any one of the preceding claims, wherein the polyethylenimine polymer has an average molecular weight of about 10 - 25 kD.

11. A pharmaceutical composition according to any one of the preceding claims, wherein the polyethylenimine polymer produces hydrophobic domains.

12. A pharmaceutical composition according to claim 11, wherein the level of hydrophobic modification is from about 0.01 - 50%, about 0.1 - 20% or about 1 - 10% of amino groups.

13. A pharmaceutical composition according to any preceding claim, wherein the polyethylenimine polymer comprises monomeric subunits in accordance with the structure as defined in formula I shown below: 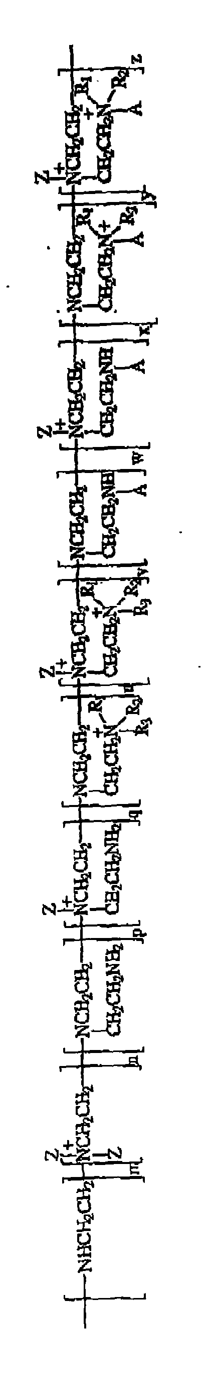 wherein
m is between 0 - 90 %;
n is between 0 - 100 %;
p is between 0 - 50 %;
q is between 0 - 50 %;
u is between 0 - 50 %;
v is between 0 - 50 %;
w is between 0 - 20 %;
x is between 0 - 20 %;
y is between 0 - 20 %; and
z is between 0 - 20 %;
wherein, m + n + p + q + u + v + w + x + y + z = 100%.

14. A pharmaceutical composition according to claim 13, wherein the monomer units m, n, p, q, u, v, w, x, y and z are arranged in any order.

15. A pharmaceutical composition according to claim 13 or claim 14, any preceding claim wherein:
when m = 0% then n is not equal to 0%;
when n = 0% then m is not equal to 0 %;
when p = 0% then q + u + v + w + x + y + z does not equal 0%;
when q = 0% then p + u + v + w + x + y + z does not equal 0%;
when u = 0% then p + q + v + w + x + y + z does not equal 0%;
when v = 0% then p + q + u + w + x + y + z does not equal 0%;
when w = 0% then x + y + z + n does not equal 0%;
when x = 0% then w + y + z + n does not equal 0%;
when y = 0% then w + x + z + n does not equal 0%;
when z = 0% then w + x + y + n does not equal zero.

16. A pharmaceutical composition according to any one of claims 13 to 15, wherein:
(a) m + n lies between 50 to 100%; and/or
(b) p + q + u + v lies between 20 to 50%; and/or
(c) wherein w + x + y + z lies between 0.01 to 10%; and/or
(d) p, q, u, v, w, x, y or z are equal to 0%; and/or
(e) the sum total of p, q, u, v, w, x, y and z is equal to a value greater than 0% thereby forming a branched compound; and/or
(f) w, x, y or z are equal to 0%; and/or
(g) m + n = 60%, w + x + y + z = 6%, and p + q + u + v = 34%.

17. A pharmaceutical composition comprising a polyethylenimine polymer according to any one of the preceding claims, further comprising a pharmaceutically acceptable carrier.

18. A pharmaceutical composition according to claim 17, wherein the weight to volume ratio (w.v.) of the polyethylenimine polymer to the pharmaceutically acceptable carrier ranges from any of the following: 0.0001 - 100 w.v., 0.005 - 50 w.v.; 0.001 - 30 w.v.; 0.001 - 10 w.v.; or 0.01 - 1 w.v.

19. A pharmaceutical composition according to claim 17 or claim 18 comprising a drug, wherein the drug is selected from any of the following: cyclosporin; steroids such as prednisolone, oestradiol, testosterone; drugs with multicyclic ring structures which lack polar groups such as paclitaxel; and drugs such as etoposide.

20. A pharmaceutical composition according to any one of claims 17 to 19, wherein the ratio of the polyethylenimine polymer to the drug is selected from any of the following: 0.001-100%; 0.1-100%; 1-100%; 10-90% or 30-70%.

21. A pharmaceutical composition according to any one of claims 17 to 20, wherein the ratio of polyethylenimine polymer to drug to pharmaceutically acceptable carrier is selected from:
(a) about 5-20 mg : 0.5-5 mg : 0.5-5 mL; or
(b) 5-20 mg : about 5-5 mg : 0.5-5 g; or
(c) about 10 mg:2 mg: 1 mL; or
(d) about 10 mg:2 mg: 2 g.

22. A pharmaceutical composition according to claim 21, wherein the pharmaceutical composition is in the form of tablets, suppositories, liquid capsule powder form or a form suitable for pulmonary delivery.

23. A pharmaceutical composition according to any one of claims 17 to 22, wherein the drug is formulated for oral delivery

24. Use of a polyethylenimine polymer as defined in any one of claims 1 to 16 for the preparation of a medicament wherein the polymer is formulated with a poorly soluble drug, wherein the drug has an aqueous solubility of less than 0.1 mg per millilitre of solvent at a temperature of about 15-20°C.

25. The use according to claim 24, wherein the poorly soluble drug is selected from any of the following: cyclosporin; steroids; drugs with multicyclic ring structures which lack polar groups; etoposide.

26. A method of forming a polyethylenimine polymer defined according to any one of claims 1 to 16, the method comprising reacting a polyethylenimine compound formed from the polymerisation of ethylenimine with a first organo halide to form an organo side chain on the polyethylenimine compound, and then reacting a second organo halide with an amino group on the polyethyleneimine polymer.

27. A method according to claim 26, wherein the ethylenimine is branched or linear.

28. A method according to claim 26 or claim 27, wherein the first organo halide is any linear or branched, substituted or unsubsituted, or cyclo form of any alkyl, alkenyl, alkynyl, aryl or acyl halide or any hydrophilic halide.

29. A method according to claim 28, wherein the first organo halide is:
(a) a C₁-C₂₀ group, a C₁-C₁₂ group, a C₁-C₆ group or a C₁ group; or
(b) a linear C₁₂-C₁₆ alkyl halide; or
(c) cetyl bromide.

30. A method according to any one of claims 26 to 29, wherein the second organo halide is a linear or branched, substituted or unsubsituted, or cyclo form of an alkyl, alkenyl, alkynyl, aryl or acyl halide or a hydrophilic halide.

31. A method according to claim 30, wherein the second organo halide is:
(a) a C₁-C₂₀ group, a C₁-C₁₂ group, a C₁-C₆ group or a C₁ group; or
(b) a linear C₁-C₆ alkyl halide; or
(c) methyl iodide.

32. A method according to any of claims 28 to 31, wherein:
(a) the polyethylenimine compound and first organo halide are mixed in an organic solvent, which is then refluxed in an alcoholic solution of sodium hydroxide, and cetyl polyethylenimine is then isolated and reacted with the second organo halide; or
(b) the second organo halide is added in the presence of a metal hydroxide, a metal halide and an alcohol.

## Patentansprüche

1. Pharmazeutische Zusammensetzung eines schwach Iöslichen Arzneistoffes und eines amphiphilen Polyethylenimin-Polymeren, wobei der Arzneistoff eine wässrige Löslichkeit von weniger als 0,1 mg pro Milliliter Lösungsmittel bei einer Temperatur von etwa 15-20 °C aufweist, wobei das Polymere hydrophile Substituenten, die quaternäre Stickstoffgruppen umfassen, und hydrophobe Substituenten der folgenden Formel enthält: 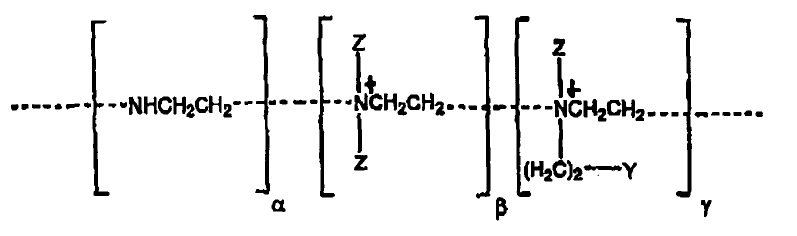 wobei
α 0 bis 90 % beträgt;
β 0 bis 100 % beträgt;
γ 0 bis 50 % beträgt;
und α + β + γ = 100%; und
wobei ferner:
die Gruppen Z jeweils unabhängig voneinander Wasserstoff oder einen hydrophoben Substituenten bedeuten, wobei es sich beim hydrophoben Substituenten um eine lineare oder verzweigte, substituierte oder unsubstituierte oder cyclische Gruppe handelt; und
Y einen hydrophilen Substituenten bedeutet.

2. Pharmazeutische Zusammensetzung nach Anspruch 1, wobei
(a) die mit α, β und γ bezeichneten Monomereinheiten eine beliebige Anordnung im Polyethylenimin-Polymeren bilden;
(b) die Anordnung der α-, β- und γ-Einheiten statistisch oder in Blockcopolymerform ist.

3. Pharmazeutische Zusammensetzung nach Anspruch 1 oder 2, wobei das Polyethylenimin-Polymere linear oder verzweigt ist.

4. Pharmazeutische Zusammensetzung nach einem der vorstehenden Ansprüche, wobei im Polyethylen-Polymeren:
(a) mindestens eine der Z-Gruppen ausgewählt ist aus: einer Alkylgruppe, einer Alkenylgruppe und Alkinylgruppe, einer Arylgruppe oder einer Acylgruppe; oder
(b) mindestens eine der Z-Gruppen ausgewählt ist aus einer C₁-C₂₀-Gruppe, einer C₁-₁₂-Gruppe, einer C₁-C₆-Gruppe oder einer C₁-Gruppe; oder
(c) mindestens eine der Z-Gruppen eine lineare C1-C4-Alkylgruppe bedeutet.

5. Pharmazeutische Zusammensetzung nach einem der vorstehenden Ansprüche, wobei im Polyethylenimin-Polymeren Y die Bedeutung -NH2, -NHA, -N⁺R₁R₂R₃ oder -N⁺R₁R₂A hat und wobei jeder der Reste R₁, R₂ oder R₃ unabhängig voneinander eine lineare oder verzweigte, substituierte oder unsubstituierte oder Cyclogruppe bedeutet, ausgewählt aus: Alkyl, Alkenyl, Alkinyl, Aryl, Acyl, Hydroxyalkyl, Hydroxyacyl, Polyethylenglycol oder ein Zucker; und A eine lineare oder verzweigte, substituierte oder unsubstituierte C₁-C₃₀-Gruppe oder Cyclogruppe bedeutet.

6. Pharmazeutische Zusammensetzung nach Anspruch 5, wobei im Polyethylenimin-Polymeren sich 1 bis 3 R₁₋₃-Substituenten an einem beliebigen einzelnen Stickstoffatom befinden.

7. Pharmazeutische Zusammensetzung nach Anspruch 5 oder 6, wobei:
(a) jeder der Reste R₁, R₂ und R₃ unabhängig voneinander eine C₁-C₂₀-Gruppe, eine C₁-C₁₂-Gruppe, eine C₁-C₆-Gruppe oder eine C₁-Gruppe bedeutet; oder
(b) jeder der Reste R₁, R₂ und R₃ eine lineare C₁-C₄-Alkylgruppe bedeutet; oder
(c) jeder der Reste R₁, R₂ und R₃ die Bedeutung CH₃ hat.

8. Zusammensetzung nach Anspruch 5, wobei im Polyethylenimin-Polymeren:
(a) die A-Gruppe eine lineare oder verzweigte, substituierte oder unsubstituierte Gruppe oder Cyclogruppen bedeutet, die aus C₁-C₃₀-, C₈-C₂₄- oder C₁₂-C₁₆-Gruppen ausgewählt sind; oder
(b) die A-Gruppe eine lineare C₁₂-C₁₆-Alkylgruppe bedeutet; oder
(c) die A-Gruppe CH₃(CH₂)₁₅ bedeutet.

9. Pharmazeutische Zusammensetzung nach einem der vorstehenden Ansprüche, wobei im Polyethylenimin-Polymeren das Verhältnis der quaternären Ammonium-Stickstoffatome zu Stickstoffatomen von Aminogruppen ausgewählt ist aus: 0,01%-100%; 10%-90%; 30%-70%; 40%-60%; 50%-90%; 60%-80% oder 40%-90%.

10. Pharmazeutische Zusammensetzung nach einem der vorstehenden Ansprüche, wobei das Polyethylenimin-Polymere ein durchschnittliches Molekulargewicht von etwa 10 bis 25 kD aufweist.

11. Pharmazeutische Zusammensetzung nach einem der vorstehenden Ansprüche, wobei das Polyethylenimin-Polymere hydrophobe Domänen erzeugt.

12. Pharmazeutische Zusammensetzung nach Anspruch 11, wobei der Grad der hydrophoben Modifikation etwa 0,01-50%, etwa 0,1-20% oder etwa 1-10% der Aminogruppen betrifft.

13. Pharmazeutische Zusammensetzung nach einem der vorstehenden Ansprüche, wobei das Polyethylenimin-Polymere monomere Untereinheiten gemäß der Struktur in der nachstehend angegebenen Formel I umfasst: 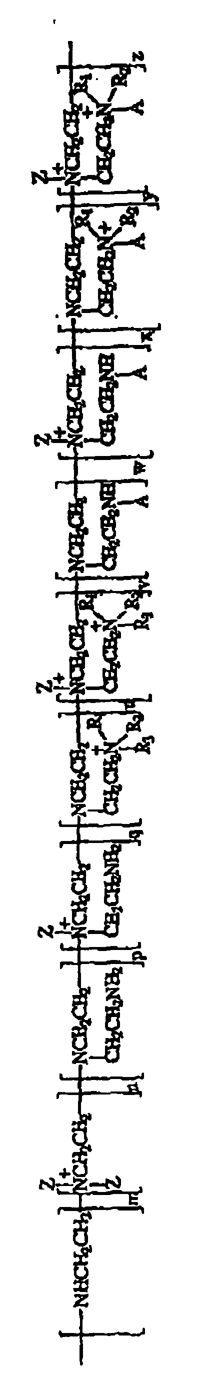 wobei
m 0 - 90 % ausmacht;
n 0 - 100 % ausmacht;
p 0 - 50 % ausmacht;
q 0 - 50 % ausmacht;
u 0 - 50 % ausmacht;
v 0 - 50 % ausmacht;
w 0 - 20 % ausmacht;
x 0 - 20 % ausmacht;
y 0 - 20 % ausmacht; und
z 0 - 20 % ausmacht;
wobei m + n + p + q + u + v + w + x + y + z = 100%

14. Pharmazeutische Zusammensetzung nach Anspruch 13, wobei die Monomereinheiten m, n, p, q, u, v, w, x, y und z in einer beliebigen Reihenfolge angeordnet sind.

15. Pharmazeutische Zusammensetzung nach Anspruch 13 oder 14, wobei:
wenn m = 0 % gilt, n nicht gleich 0 % ist;
wenn n = 0 % gilt, m nicht gleich 0 % ist;
wenn p = 0 % gilt, q + u + v + w + x + y + z nicht gleich 0 % sind;
wenn q = 0 % gilt, p + u + v + w + x + y + z nicht gleich 0 % sind;
wenn u = 0 % gilt, p + q + v + w + x + y + z nicht gleich 0 % sind;
wenn v = 0 % gilt, p + q + u + w + x + y + z nicht gleich 0 % sind;
wenn w = 0 % gilt, x + y + z + n nicht gleich 0 % sind;
wenn x = 0 % gilt, w + y + z + n nicht gleich 0 % sind;
wenn y = 0 % gilt, w + x + z + n nicht gleich 0 % sind;
wenn z = 0 % gilt, w + x + y + n nicht gleich 0 % sind.

16. Pharmazeutische Zusammensetzung nach einem der Ansprüche 13 bis 15, wobei
(a) m + n 50 bis 100 % betragen; und/oder
(b) p + q + u + v 20 bis 50 % betragen; und/oder
(c) wobei w + x + y + z 0,01 bis10 % betragen; und/oder
(d) p, q, u, v, w, x, y oder z 0 % betragen; und/oder
(e) die Summe aus p, q, u, v, w, x, y und z einen Wert ergeben, der größer als 0 % ist, wodurch eine verzweigte Verbindung gebildet wird; und/oder
(f) w, x, y oder z 0 % ergeben; und/oder
(g) m + n = 60%, w + x + y + z = 6% und p + q + u + v = 34%.

17. Pharmazeutische Zusammensetzung, umfassend ein Polyethylenimin-Polymeres nach einem der vorstehenden Ansprüche, ferner umfassend einen pharmazeutisch verträglichen Träger.

18. Pharmazeutische Zusammensetzung nach Anspruch 17, wobei das Verhältnis von Gewicht/Volumen (w.v.) des Polyethylenimin-Polymeren zum pharmazeutisch verträglichen Träger in einem der folgenden Bereiche liegt: 0,0001-100 w.v., 0,005-50 w.v., 0,001-30 w.v., 0,001-10 w.v. oder 0,01-1 w.v.

19. Pharmazeutische Zusammensetzung nach Anspruch 17 oder 18, umfassend einen Arzneistoff, wobei der Arzneistoff in beliebiger Weise aus der folgenden Gruppe ausgewählt ist: Cyclosporin; Steroide, wie Prednisolon, Östradiol, Testosteron; Arzneistoffe mit multicyclischen Ringstrukturen, bei denen polare Gruppen fehlen, wie Paclitaxel; und Arzneistoffe, wie Etoposid.

20. Pharmazeutische Zusammensetzung nach einem der Ansprüche 17 bis 19, wobei das Verhältnis des Polyethylenimin-Polymeren zum Arzneistoff in beliebiger Weise aus einem der folgenden Bereiche ausgewählt ist: 0,001-100%; 0,1-100%, 1-100%; 10-90% oder 30-70%.

21. Pharmazeutische Zusammensetzung nach einem der Ansprüche 17 bis 20, wobei das Verhältnis vom Polyethylenimin-Polymeren zum Arzneistoff und dem pharmazeutisch verträglichen Träger ausgewählt ist aus:
(a) etwa 5-20 mg : 0,05-5 mg : 0,5-5 ml; oder
(b) 5-20 mg : etwa 5-5 mg : 0,5-5 g; oder
(c) etwa 10 mg : 2 mg : 1 ml; oder
(d) etwa 10 mg : 2 mg : 2 g.

22. Pharmazeutische Zusammensetzung nach Anspruch 21, wobei die pharmazeutische Zusammensetzung in Form von Tabletten, Suppositorien, flüssigkeitsgefüllten Kapseln, in Pulverform oder in einer Form, die für die pulmonale Abgabe geeignet ist, vorliegt.

23. Pharmazeutische Zusammensetzung nach einem der Ansprüche 17 bis 22, wobei der Arzneistoff für die orale Abgabe zubereitet ist.

24. Verwendung eines Polyethylenimin-Polymeren gemäß einem der Ansprüche 1 bis 16 zur Herstellung eines Arzneimittels, wobei das Polymere mit einem schwach löslichen Arzneistoff zubereitet wird, wobei der Arzneistoff eine wässrige Löslichkeit von weniger als 0,1 mg pro Milliliter Lösungsmittel bei einer Temperatur von etwa 15-20 °C aufweist.

25. Verwendung nach Anspruch 24, wobei der schwach lösliche Arzneistoff aus einem beliebigen der folgenden Arzneistoffe ausgewählt wird: Cyclosporin; Steroide; Arzneistoffe mit multicyclischen Ringstrukturen, bei denen polare Gruppen fehlen; Etoposid.

26. Verfahren zur Bildung eines Polyethylenimin-Polymeren gemäß einem der Ansprüche 1 bis 16, wobei das Verfahren folgendes umfasst: Umsetzung einer Polyethylenimin-Verbindung, die durch Polymerisation von Ethylenimin mit einem ersten Organohalogenid unter Bildung einer Organoseitenkette an der Polyethylenimin-Verbindung gebildet worden ist, und anschließende Umsetzung eines zweiten Organohalogenids mit einer Aminogruppe am Polyethylenimin-Polymeren.

27. Verfahren nach Anspruch 26, wobei das Ethylenimin verzweigt oder linear ist.

28. Verfahren nach Anspruch 26 oder 27, wobei es sich beim ersten Organohalogenid um ein beliebiges lineares oder verzweigtes, substituiertes oder unsubstituiertes oder cyclisches Alkyl-, Alkenyl-, Alkinyl-, Aryl- oder Acylhalogenid oder um ein beliebiges hydrophiles Halogenid handelt.

29. Verfahren nach Anspruch 28, wobei es sich beim ersten Organohalogenid um:
(a) eine C₁-C₂₀-Gruppe, eine C₁-C₁₂-Gruppe, eine C₁-C₆-Gruppe oder eine C₁-Gruppe; oder
(b) ein lineares C₁₂-C₁₆-Alkylhalogenid; oder
(c) Cetylbromid handelt.

30. Verfahren nach einem der Ansprüche 26 bis 29, wobei es sich beim zweiten Organohalogenid um eine lineares oder verzweigtes, substituiertes oder unsubstituiertes oder cyclisches Alkyl-, Alkenyl-, Alkinyl-, Aryl- oder Acylhalogenid oder um ein hydrophiles Halogenid handelt.

31. Verfahren nach Anspruch 30, wobei es sich beim zweiten Organohalogenid um
(a) eine C₁-C₂₀-Gruppe, eine C₁-C₁₂-Gruppe, eine C₁-C₆-Gruppe oder eine C₁-Gruppe; oder
(b) ein lineares C₁-C₆-Alkylhalogenid; oder
(c) Methyljodid handelt.

32. Verfahren nach einem der Ansprüche 28 bis 31, wobei:
(a) die Polyethylenimin-Verbindung und das erste Organohalogenid in einem organischen Lösungsmittel vermischt werden, das anschließend in einer alkoholischen Lösung von Natriumhydroxid unter Rückfluss erwärmt wird, wobei anschließend das Cetylpolyethylenimin isoliert und mit dem zweiten Organohalogenid umgesetzt wird; oder
(b) das zweite Organohalogenid in Gegenwart eines Metallhydroxids, eines Metallhalogenids und eines Alkohols zugesetzt wird.

## Revendications

1. Composition pharmaceutique d'un médicament médiocrement soluble et d'un polymère de polyéthylèneimine amphiphile, dans laquelle le médicament présente une solubilité aqueuse inférieure à 0,1 mg par millilitre de solvant à une température d'environ 15-20°C, dans laquelle le polymère contient des substituants hydrophiles qui comprennent des groupes d'azote quaternaire et des substituants hydrophobes comme représenté par la formule : 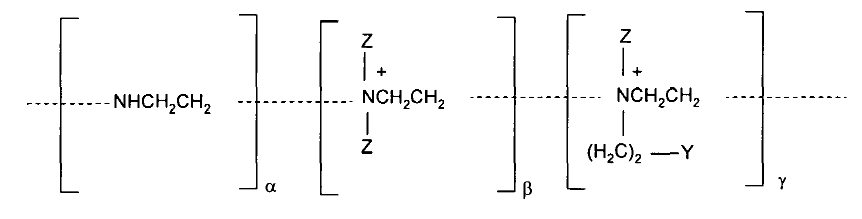 dans laquelle
α est compris entre 0 et 90 % ;
β est compris entre 0 et 100 % ;
γ est compris entre 0 et 50 % ;
et α + β + γ = 100 % ; et
et de plus dans laquelle :
chaque groupe Z est indépendamment un atome d'hydrogène ou un substituant hydrophobe, dans laquelle le substituant hydrophobe est un groupe linéaire ou ramifié, substitué ou non substitué ou un groupe cyclique ; et
Y est un substituant hydrophile.

2. Composition pharmaceutique selon la revendication 1, dans laquelle :
(a) les unités monomères identifiées avec α, β et γ forment un arrangement quelconque dans le polymère de polyéthylèneimine ;
(b) l'arrangement des α, β et γ unités est aléatoire ou dans une forme de copolymère séquencé.

3. Composition pharmaceutique selon la revendication 1 ou la revendication 2, dans laquelle le polymère de polyéthylèneimine est linéaire ou ramifié.

4. Composition pharmaceutique selon l'une quelconque des revendications précédentes, dans laquelle dans le polymère de polyéthylèneimine :
(a) au moins un des groupes Z est choisi parmi :
un groupe alkyle, un groupe alcényle, un groupe alcynyle, un groupe aryle ou un groupe acyle ; ou
(b) au moins un des groupes Z est choisi parmi un groupe en C₁-C₂₀, un groupe en C₁-C₁₂, un groupe en C₁-C₆ ou un groupe en C₁ ; ou
(c) au moins un des groupes Z est un groupe alkyle linéaire en C₁-C₄.

5. Composition pharmaceutique selon l'une quelconque des revendications précédentes, dans laquelle dans le polymère de polyéthylèneimine, Y représente -NH₂, -NHA, -N⁺R₁R₂R₃ ou -N⁺R₁R₂A, et dans laquelle chacun des R₁, R₂ ou R₃ est indépendamment un groupe linéaire ou ramifié, substitué ou non substitué, ou un groupe cyclo choisi parmi : un groupe alkyle, alcényle, alcynyle, aryle, acyle, hydroxyalkyle, hydroxyacyle, polyéthylèneglycol ou un sucre ; et A est un groupe linéaire ou ramifié, substitué ou non substitué ou un groupe cyclo en C₁-C₃₀,

6. Composition pharmaceutique selon la revendication 5, dans laquelle dans le polymère de polyéthylèneimine, il y a entre 1 et 3 substitutants R₁₋₃ sur tout azote unique.

7. Composition pharmaceutique selon la revendication 5 ou la revendication 6, dans laquelle :
(a) chacun parmi R₁, R₂ et R₃ est indépendamment un groupe en C₁-C₂₀, un groupe en C₁-C₁₂, un groupe en C₁-C₆ ou un groupe en C₁ ; ou
(b) chacun parmi R₁, R₂ et R₃ est un groupe alkyle linéaire en C₁-C₄ ; ou
(c) chacun parmi R₁, R₂ et R₃ est CH₃.

8. Composition pharmaceutique selon la revendication 5, dans laquelle dans le polymère de polyéthylèneimine :
(a) le groupe A est un groupe linéaire ou ramifié, substitué ou non substitué ou un groupe cyclo choisi parmi un groupe en C₁-C₃₀, un groupe en C₈-C₂₄ ou un groupe en C₁₂-C₁₆ ; ou
(b) le groupe A est un groupe alkyle en C₁₂-C₁₆ linéaire ; ou
(c) le groupe A est CH₃(CH₂)₁₅.

9. Composition pharmaceutique selon l'une quelconque des revendications précédentes, dans laquelle dans le polymère de polyéthylèneimine, le rapport des azotes d'ammonium quaternaire aux azotes des groupes amino est choisi parmi : 0,01 % - 100 %, 10 % - 90 % ; 30 % - 70 % ; 40 % - 60 % ; 50 - 90 % ; 60 % - 80 % ou 40 % - 90 %.

10. Composition pharmaceutique selon l'une quelconque des revendications précédentes, dans laquelle le polymère de polyéthylèneimine présente une masse moléculaire moyenne d'environ 10-25 kD.

11. Composition pharmaceutique selon l'une quelconque des revendications précédentes, dans laquelle le polymère de polyéthylèneimine produit des domaines hydrophobes.

12. Composition pharmaceutique selon la revendication 11, dans laquelle le niveau de modification hydrophobe est d'environ 0,01 - 50 %, d'environ 0,1-20 % ou d'environ 1 - 10 % de groupes amino.

13. Composition pharmaceutique selon l'une quelconque des revendications précédentes, dans laquelle le polymère de polyéthylèneimine comprend des sous-unités monomères selon la structure comme défini dans la formule (I) représentée ci-dessus : 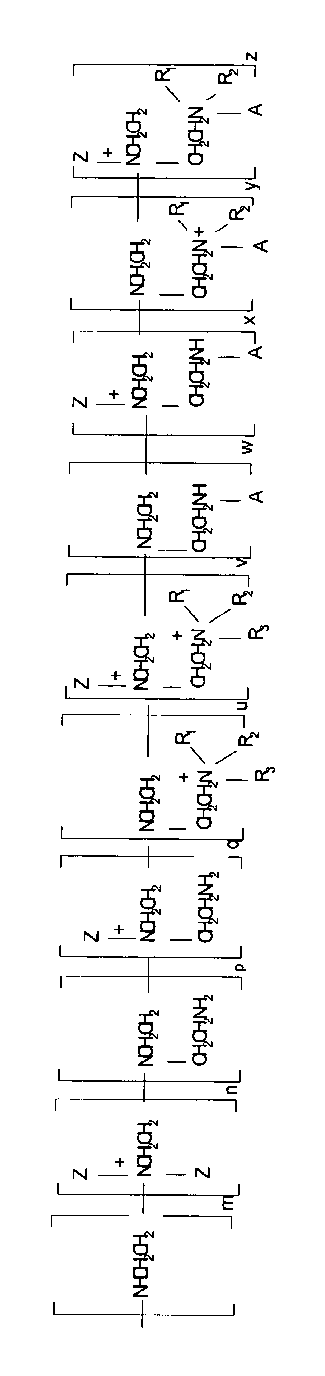 dans laquelle
m est compris entre 0 et 90 % ;
n est compris entre 0 et 100 % ;
p est compris entre 0 et 50 % ;
q est compris entre 0 et 50 % ;
u est compris entre 0 et 50 % ;
v est compris entre 0 et 50 % ;
w est compris entre 0 et 20 % ;
x est compris entre 0 et 20 % ;
y est compris entre 0 et 20 % ; et
z est compris entre 0 et 20 % ;
dans laquelle m + n + p + q + u + v + w + x + y + z = 100 %.

14. Composition pharmaceutique selon la revendication 13, dans laquelle les unités monomères m, n, p, q, u, v, w, x, y et z sont disposées dans un ordre quelconque.

15. Composition pharmaceutique selon la revendication 13 ou la revendication 14, une revendication précédente quelconque, dans laquelle :
lorsque m est égal à 0 % alors n n'est pas égal à 0 % ;
lorsque n est égal à 0 % alors m n'est pas égal à 0 % ;
lorsque p est égal à 0 % alors q + u + v + w + x + y + z n'est pas égal à 0 % ;
lorsque q est égal à 0 % alors p + u + v + w + x + y + z n'est pas égal à 0 % ;
lorsque u est égal à 0 % alors p + q + v + w + x + y + z n'est pas égal à 0 % ;
lorsque v est égal à 0 % alors p + q + u + w + x + y + z n'est pas égal à 0 % ;
lorsque w est égal à 0 % alors x + y + z + n n'est pas égal à 0%;
lorsque x est égal à 0 % alors w + y + z + n n'est pas égal à 0%;
lorsque y est égal à 0 % alors w + x + z + n n'est pas égal à 0%;
lorsque z est égal à 0 % alors w + x + y + n n'est pas égal à 0%;

16. Composition pharmaceutique selon l'une quelconque des revendications 13 à 15, dans laquelle :
(a) m + n est compris entre 50 et 100 % ; et/ou
(b) p + q + u + v est compris entre 20 et 50 % ; et/ou
(c) dans laquelle w + x + y + z est compris entre 0,01 et 10 % ; et/ou
(d) p, q, u, v, w, x, y ou z sont égaux à 0 % ; et/ou
(e) la somme totale de p, q, u, v, w, x, y et z est égale à une valeur supérieure à 0 % formant par là un composé ramifié ; et/ou
(f) w, x, y ou z sont égaux à 0 % ; et/ou
(g) m + n = 60 %, w + x + y + z = 6 %, et p + q + u + v = 34 %.

17. Composition pharmaceutique comprenant un polymère de polyéthylèneimine selon l'une quelconque des revendications précédentes, comprenant de plus un support pharmaceutiquement acceptable.

18. Composition pharmaceutique selon la revendication 17, dans laquelle le rapport masse à volume (m.v.) du polymère de polyéthylèneimine au support pharmaceutiquement acceptable est compris dans l'un des intervalles suivants : 0,0001 - 100 m.v., 0,005 - 50 m.v. ; 0,001 - 30 m.v. ; 0,001 - 10 m.v. ; ou 0,01 - 1 m.v.

19. Composition pharmaceutique selon la revendication 17 ou la revendication 18 comprenant un médicament, dans laquelle le médicament est choisi parmi l'un quelconque des suivants : la cyclosporine ; des stéroïdes, tels que la prednisolone, l'oestradiol, la testostérone ; des médicaments avec des structures de noyaux multicycliques qui manquent de groupes polaires, tels que le paclitaxel ; et des médicaments tels que l'étoposide.

20. Composition pharmaceutique selon l'une quelconque des revendications 17 à 19, dans laquelle le rapport du polymère de polyéthylèneimine au médicament est choisi parmi l'un quelconque des suivants : 0,001 - 100 % ; 0,1 - 100 % ; 1 - 100 % ; 10-90 % ou 30-70%.

21. Composition pharmaceutique selon l'une quelconque des revendications 17 à 20, dans laquelle le rapport du polymère de polyéthylèneimine au médicament au support pharmaceutiquement acceptable est choisi parmi :
(a) environ 5 - 20 mg : 0,05 - 5 mg : 0,5 - 5 ml ; ou
(b) 5 - 20 mg : environ 5 - 5 mg : 0,5 - 5 g ; ou
(c) environ 10 mg : 2 mg : 1 ml ; ou
(d) environ 10 mg : 2 mg : 2 g.

22. Composition pharmaceutique selon la revendication 21, dans laquelle la composition pharmaceutique est dans la forme de comprimés, de suppositoires, dans une forme de poudre en capsule liquide ou dans une forme appropriée pour une administration pulmonaire.

23. Composition pharmaceutique selon l'une quelconque des revendications 17 à 22, dans laquelle le médicament est formulé pour une administration orale.

24. Utilisation d'un polymère de polyéthylèneimine selon l'une quelconque des revendications 1 à 16 pour la préparation d'un médicament dans lequel le polymère est formulé avec un médicament médiocrement soluble, dans laquelle le médicament présente une solubilité aqueuse inférieure à 0,1 mg par millilitre de solvant à une température d'environ 15-20°C.

25. Utilisation selon la revendication 24, dans laquelle le médicament médiocrement soluble est choisi parmi l'un quelconque des suivants : la cyclosporine ; des stéroïdes ; des médicaments avec des structures de noyaux multicycliques qui manquent de groupes polaires ; l'étoposide.

26. Procédé de formation d'un polymère de polyéthylèneimine défini selon l'une quelconque des revendications 1 à 16, le procédé comprenant la réaction d'un composé de polyéthylèneimine formé à partir de la polymérisation d'éthylèneimine avec un premier organohalogénure pour former une chaîne latérale organo sur le composé de polyéthylèneimine et la réaction subséquente d'un second organohalogénure avec un groupe amino sur le polymère de polyéthylèneimine.

27. Procédé selon la revendication 26, dans lequel l'éthylèneimine est ramifiée ou linéaire.

28. Procédé selon la revendication 26 ou la revendication 27, dans lequel le premier organohalogénure est une forme linéaire ou ramifiée, substituée ou non substituée ou cyclo d'un halogénure quelconque d'un halogénure d'alkyle, d'alcényle, d'alcynyle, d'aryle ou d'acyle quelconque ou d'un halogénure hydrophile quelconque.

29. Procédé selon la revendication 28, dans lequel le premier organohalogénure est :
(a) un groupe en C₁-C₂₀, un groupe en C₁-C₁₂, un groupe en C₁-C₆ ou un groupe en C₁ ; ou
(b) un halogénure d'alkyle en C₁₂-C₁₆ linéaire ; ou
(c) le bromure de cétyle.

30. Procédé selon l'une quelconque des revendications 26 à 29, dans lequel le second organohalogénure est une forme linéaire ou ramifiée, substituée ou non substituée, ou cyclo d'un halogénure d'alkyle, d'alcényle, d'alcynyle, d'aryle ou d'acyle ou d'un halogénure hydrophile.

31. Procédé selon la revendication 30, dans lequel le second organohalogénure est :
(a) un groupe en C₁-C₂₀, un groupe en C₁-C₁₂, un groupe en C₁-C₆ ou un groupe en C₁ ; ou
(b) un halogénure d'alkyle en C₁-C₆ linéaire ; ou
(c) l'iodure de méthyle.

32. Procédé selon l'une quelconque des revendications 28 à 31, dans lequel :
(a) le composé de polyéthylèneimine et le premier organohalogénure sont mélangés dans un solvant organique, lequel est ensuite mis sous reflux dans une solution alcoolique d'hydroxyde de sodium, et la cétylpolyéthylèneimine est ensuite isolée et réagit avec le second organohalogénure ; ou
(b) le second organohalogénure est ajouté en présence d'un hydroxyde de métal, d'un halogénure de métal et d'un alcool.
